(19) Europäisches Patentamt
European Patent Office
Office européen des brevets

(11) **EP 3 331 449 B1**

(12) **EUROPEAN PATENT SPECIFICATION**

(45) Date of publication and mention
of the grant of the patent:
**19.12.2018 Bulletin 2018/51**

(51) Int Cl.:
*A61B 6/03* (2006.01)  *A61B 6/00* (2006.01)
*G06T 11/00* (2006.01)

(21) Application number: **16781409.4**

(22) Date of filing: **12.10.2016**

(86) International application number:
**PCT/EP2016/074456**

(87) International publication number:
**WO 2017/071956 (04.05.2017 Gazette 2017/18)**

(54) **COMPUTED TOMOGRAPHY IMAGE GENERATION APPARATUS**

COMPUTERTOMOGRAFIEBILDERZEUGUNGSVORRICHTUNG

APPAREIL DE GÉNÉRATION D'IMAGE DE TOMOGRAPHIE ASSISTÉE PAR ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2015 EP 15191838**

(43) Date of publication of application:
**13.06.2018 Bulletin 2018/24**

(73) Proprietor: **Koninklijke Philips N.V.**
**5656 AE Eindhoven (NL)**

(72) Inventors:
• **GRASS, Michael**
  **5656 AE Eindhoven (NL)**
• **HOMAN, Robert, Johannes, Frederik**
  **5656 AE Eindhoven (NL)**
• **PROKSA, Roland**
  **5656 AE Eindhoven (NL)**

(74) Representative: **van Iersel, Hannie**
**Philips Intellectual Property & Standards**
**High Tech Campus 5**
**5656 AE Eindhoven (NL)**

(56) References cited:
• **BODENSTEINER C ET AL: "Motion and Positional Error Correction for Cone Beam 3D-Reconstruction with Mobile C-Arms", 29 October 2007 (2007-10-29), MEDICAL IMAGE COMPUTING AND COMPUTER-ASSISTED INTERVENTION - MICCAI 2007; [LECTURE NOTES IN COMPUTER SCIENCE], SPRINGER BERLIN HEIDELBERG, BERLIN, HEIDELBERG, PAGE(S) 177 - 185, XP019081681, ISBN: 978-3-540-75756-6 p. 178, section 2 - p. 184, section 4; figures 2, 4**
• **Hanno Schumacher ET AL: "A New Approach for Motion Correction in SPECT Imaging" In: "Bildverarbeitung fuer die Medizin 2007. Proceedings des Workshops vom 25.-27. März 2007 in München", 1 January 2007 (2007-01-01), Springer, XP055262921, ISBN: 978-3-540-71090-5 pages 51-55, DOI: 10.1007/978-3-540-71091-2_11, page 52 - page 53**
• **LEE K J AND BARBER D C: "Use of forward projection to correct patient motion during SPECT imaging", PHYSICS IN MEDICINE AND BIOLOGY, INSTITUTE OF PHYSICS PUBLISHING, BRISTOL GB, vol. 43, 1 January 1998 (1998-01-01), pages 171-187, XP002330937, ISSN: 0031-9155, DOI: 10.1088/0031-9155/43/1/011**

**Description**

FIELD OF THE INVENTION

The invention relates to a computed tomography image generation apparatus and method for generating an image of a human head. The invention relates further to a computer program for controlling the computed tomography image generation apparatus.

BACKGROUND OF THE INVENTION

The article "Motion and Positional Error Correction for Cone Beam 3D-Reconstruction with Mobile C-Arms" by C. Bodensteiner et al., Medical Image Computing and Computer-Assisted Intervention, volume 4791, pages 177-185 (2007) discloses a mobile C-arm device for acquiring computed tomography images. The C-arm device is configured to perform iterative three-dimensional reconstruction and two-dimensional/three-dimensional registration algorithms for correcting projection data inconsistencies, wherein the projection data inconsistencies are caused by positioning errors of the C-arm device.

Computed tomography image generation apparatuses for generating an image of a human head comprise a reconstruction unit for reconstructing the image based on acquired projections. The projections depend on radiation, which has traversed the head and which is emitted by a radiation source moving around the head along, for instance, a circular or helical trajectory. Patients like acute stroke patients may not be able to avoid motion during the acquisition of the projections such that the reconstructed image of the head may comprise motion artifacts.

SUMMARY OF THE INVENTION

It is an object of the present invention to provide a computed tomography image generation apparatus and method for generating an image of a human head which has less motion artifacts. It is a further object of the present invention to provide a computer program for controlling the computed tomography image generation apparatus in accordance with the computed tomography image generation method.

In a first aspect of the present invention a computed tomography image generation apparatus for generating an image of a human head is presented, wherein the computed tomography image generation apparatus comprises:

- a projections providing unit for providing measured two-dimensional projections of the head, wherein the measured projections have been measured at different times while a radiation source, which emits radiation for traversing the head, has been moved around the head and wherein the measured projections have been generated based on the radiation after having traversed the head,
- a reconstruction unit for reconstructing a three-dimensional first computed tomography image of the head based on the provided measured projections,
- a transformation determination unit for determining three-dimensional transformations of the first computed tomography image of the head for different measured projection groups, wherein a measured projection group comprises one or several measured projections, wherein the transformation determination unit is adapted to determine for a certain measured projection group a transformation such that a degree of similarity between the certain measured projection group and a calculated projection group is increased, wherein the calculated projection group corresponds to the certain measured projection group and is calculated by transforming the first computed tomography image in accordance with the transformation to be determined and by forward projecting the transformed first computed tomography image,

wherein the reconstruction unit is adapted to reconstruct a motion corrected three-dimensional second computed tomography image based on the measured projections and the transformations determined for the different measured projection groups,
wherein the computed tomography image generation apparatus comprises an examination zone in which the human head is arrangeable, wherein the reconstruction unit is adapted to reconstruct the first computed tomography image and the second computed tomography image such that they show the examination zone and to, for generating the second computed tomography image, perform the motion correction for a first part of the examination zone and to perform the motion correction not for a second part of the examination zone, wherein the first part of the examination zone includes the human head.

Since the measured projections have been measured at different times and since for each measured projection group, which comprises one or several measured projections, a transformation of the first computed tomography image of the head is determined such that a degree of similarity between the respective measured projection group and the corresponding calculated projection group is increased, especially optimized, wherein the corresponding calculated projection group is determined by transforming the first computed tomography image in accordance with the transformation to be determined and by forward projecting the transformed first computed tomography image, for different times transformations of the first computed tomography image are determined, which transform the first computed tomography image such that it is in accordance with the respective measured projection group. The transformations determined for the different measured projection groups and hence for the different times therefore accurately describe the motion of the head and can be used for reconstructing a motion corrected three-dimensional second computed tomography image having only few motion artifacts or no motion artifacts at all.

A measured projection group can comprise a single measured projection or several measured projections. If a measured projection group comprises several measured projections, these measured projections are preferentially temporally adjacent. These measured projections of a same measured projection group are thereby also angularly adjacent with respect to a rotational angle describing the rotation of the radiation source around the head, i.e. a measured projection group comprising several measured projections can therefore also be regarded as comprising an angular subset of the measured projections.

The projections providing unit can be a storing unit for storing measured projections and for providing the stored measured projections. The projections providing unit can also be a receiving unit for receiving the measured projections from a projections acquisition device and for providing the received measured projections. Moreover, the projections providing unit can also be the projections acquisition device itself. The projections acquisition device may comprise a radiation source for emitting radiation, a detection device for detecting the radiation after having traversed the human head and for generating the measured projections based on the traversed radiation and means for moving the radiation source and the human head relative to each other for generating the measured projections in different acquisition directions.

The projections providing unit is preferentially adapted to provide measured projections which have been measured by using a step-and-shoot acquisition scheme or a helical acquisition scheme. This allows generating the first and second computed tomography images such that they cover a relatively large part of the head, especially the entire head, by using a detection unit having a relatively small extension parallel to a rotational axis of the rotational movement of the radiation source around the head.

A calculated projection corresponds to a measured projection, if they have been calculated and measured, respectively, in the same acquisition geometry, wherein the acquisition geometry defines the geometry of the rays of the generated, real radiation used for generating the measured projection and the geometry of the rays of the simulated radiation used for generating the calculated projection. If a calculated projection group and a measured projection group correspond to each other, they have the same number of projections and each projection of the measured projection group has a corresponding projection in the calculated projection group.

The transformation determination unit is preferentially adapted to determine rigid transformations for transforming the first computed tomography image. The transformation determination unit can be adapted to determine a transformation for a measured projection group iteratively. Moreover, in an embodiment the transformation determination unit is adapted to use a gradient angle difference for determining the degree of similarity. In particular, the transformation determination unit is adapted to use a normalized gradient angle difference for determining the degree of similarity. However, the transformation determination unit can also be adapted to use other similarity measures for determining the degree of similarity like a sum of squared differences, a gradient correlation measure, a pattern intensity measure, et cetera.

The reconstruction unit can be adapted to reduce motion artifacts in the first computed tomography image by using overscan weighting. By using overscan weighting a low level motion artifacts reduction can be provided such that for determining the transformations a first computed tomography image can be used having an improved image quality. This can lead to an improved determination of the transformations and hence to an improved motion correction when reconstructing the second computed tomography image.

In an embodiment the transformation determination unit is adapted such that each measured projection group comprises a single measured projection. If each measured projection group comprises a single measured projection only, for each single measured projection a transformation is determined such that the motion of the head can be characterized by the determined sequence of transformations with a relatively high temporal resolution.

In a further embodiment the transformation determination unit is adapted such that each measured projection group comprises several measured projections. Thus, in an embodiment several measured projections are used for determining a respective transformation. This can lead to a more accurate determination of the transformations for the different measured projection groups, which describe the motion of the head, and hence to a further improved correction of the motion artifacts in the second computed tomography image which is reconstructed based on the measured projections and the determined transformations.

The transformation determination unit can be adapted to filter the determined transformations. For instance, the transformation determination unit can be adapted to use a smoothing filter, which might be a median filter or another filter, for smoothing the determined transformations.

In an embodiment the transformation determination unit is adapted to determine transformations based on the degree of similarity and the forward projection not for all times, at which projections have been measured, and to determine transformations for times, at which transformations have not been determined based on the degree of similarity and the forward projection, by interpolation. This has the advantage that the computational efforts for determining the transformations can be reduced. Moreover, the transformation determination unit can be adapted to determine an outlier in the determined transformations and to remove the determined outlier from the determined transformations. By removing the determined outlier from the determined transformations it can be ensured that the outlier is not used for motion correction while reconstructing the second computed tomography image. This can further improve the quality of the reconstructed second computed tomography image. The removed outlier transformation may be replaced by a transformation which has been determined by interpolation, wherein an interpolation algorithm may be applied to temporally neighboring transformations. The replacing transformation may also be determined in another way, for instance, by determining the median of the temporally neighboring transformations. For determining the replacing transformation one or several transformations corresponding to earlier times and one or several transformations corresponding to later times may be used.

The interpolation and/or filtering algorithms are preferentially applied to parameters of the transformations along the temporal axis. For instance, if the transformations include three translational parameters defining a translation, the interpolation and/or filtering algorithms are applied to corresponding translational parameters of different transformations, i.e. which have been determined for different times. The transformations preferentially only include translations and rotations. However, they can also include a deformation.

The computed tomography image generation apparatus comprises an examination zone in which the human head is arrangable, wherein the reconstruction unit is adapted to reconstruct the first computed tomography image and the second computed tomography image such that they show the examination zone, wherein in an embodiment the reconstruction unit is further adapted to, for generating the second image computed tomography image, perform the motion correction for a first part of the examination zone and to perform the motion correction not for a second part of the examination zone, wherein the first part of the examination zone includes the human head. This can reduce the computational efforts for reconstructing the second computed tomography image. The first part and the second part of the examination zone can be predefined. For instance, the part of the examination zone, which is known to include a patient table and an area below the patient table, can be predefined as being the second part, whereas the first part can be the remaining part of the examination zone. It is also possible that the reconstruction unit determine the first part of the examination zone including the human head based on the first computed tomography image by using, for instance, known segmentation algorithms for segmenting the human head in the first computed tomography image. The first part could then include the segmented human head and a predefined margin surrounding the segmented human head. The predefined margin can ensure that really the complete human head is within the first part, even if the human head moves.

In an embodiment the reconstruction unit is adapted to divide the measured projections into several sets of measured projections, wherein each set includes temporally adjacent measured projections, and to reconstruct several three-dimensional first computed tomography images of the head based on the several sets of measured projections, wherein a respective first computed tomography image is reconstructed based on a respective set of measured projections. In this embodiment the transformation determination unit is adapted to determine for each first computed tomography image a set of three-dimensional transformations of the respective first computed tomography image of the head for different measured projection groups, wherein a measured projection group comprises one or several measured projections of the respective set of measured projections, wherein the transformation determination unit is adapted to determine for a certain measured projection group of the respective set of measured projections a transformation such that a degree of similarity between the certain measured projection group and a calculated projection group is increased, wherein the calculated projection group corresponds to the certain measured projection group and is calculated by transforming the respective first computed tomography image in accordance with the transformation to be determined and by forward projecting the transformed respective first computed tomography image. The reconstruction unit can then be adapted to reconstruct a motion corrected three-dimensional second computed tomography image based on the measured projections and the several sets of transformations determined for the different measured projection groups of the several sets of measured projections. Since not all measured projections are used for reconstructing a respective first computed tomography image, the measured projections used for reconstructing the respective first computed tomography image cover a relatively small time period such that the respective reconstructed first computed tomography image comprises less motion artifacts. Moreover, since these first computed tomography images showing less motion artifacts are used for determining the transformations, the quality of the transformations and hence of the finally reconstructed motion corrected second computed tomography image can be improved. The transformation determination unit can be adapted to determine a further transformation transforming a transformed first computed tomography image, which has been reconstructed based on a set of measured projections and which has then be transformed, such that it corresponds to another transformed first computed tomography image, which has been reconstructed based on another set of measured projections and which has then be transformed, wherein the reconstruction unit can be adapted to reconstruct the motion corrected three-dimensional second computed tomography image also based on the further transformation. The further transformation is preferably a registration transformation registering the transformed first computed tomography images onto each other. Preferably, several further transformations are determined for several pairs of transformed reconstructed first computed tomography images, which have been reconstructed based on neighboring measured projection groups which correspond to different sets of measured projections. By using these further transformations the quality of the finally reconstructed second computed tomography image can be further improved.

The reconstruction unit can be adapted to divide the measured projections into several sets of measured projections such that at least one set of measured projections does not fulfill the completeness criterion for computed tomography reconstruction. The completeness criterion for computed tomography reconstruction defines that projection rays covering an angular range of 180 degrees should be available for every voxel of the respective first computed tomography image to be reconstructed. Since at least one set of the measured projections does not fulfil this completeness criterion, the measured projections of this set may correspond to an angular range of, for instance, 20 degrees, 30 degrees or 40 degrees of a rotation of the radiation source around the head. In an embodiment the reconstructing unit is adapted to divide the measured projections into the several sets of measured projections such that each set of measured projections does not fulfill the completeness criterion. For instance, the measured projections can be divided into sets of measured projections, wherein a first set corresponds to an angular range from 0 degrees to 20 degrees, a second set corresponds to an angular range from 21 degrees to 40 degrees, a third set corresponds to an angular range from 41 degrees to 60 degrees et cetera, wherein these angular ranges refer to the rotational position of the radiation source while rotating around the head.

In an embodiment the reconstruction unit and the transformation determination unit are adapted such that, after the second computed tomography image has been reconstructed, in an iteration step the transformations are determined again based on the second computed tomography image, which then has the function of the first computed tomography image, and the second computed tomography image is again reconstructed based on the newly determined transformations and the measured projections. This iteration can be repeated, until an abort criterion is fulfilled. This can lead to a further improved quality of the finally generated second computed tomography image.

In a further aspect of the present invention a computed tomography image generation method for generating an image of a human head is presented, wherein the computed tomography image generation method comprises:

- providing measured two-dimensional projections of the head by a projections providing unit, wherein the measured projections have been measured at different times while a radiation source, which emits radiation for traversing the head, has been moved around the head and wherein the measured projections have been generated based on the radiation after having traversed the head,
- reconstructing a three-dimensional first computed tomography image of the head based on the provided measured projections by a reconstruction unit,
- determining three-dimensional transformations of the first computed tomography image of the head for different measured projection groups by a transformation determination unit, wherein a measured projection group comprises one or several measured projections, wherein the transformation determination unit determines for a certain measured projection group a transformation such that a degree of similarity between the certain measured projection group and a calculated projection group is increased, wherein the calculated projection group corresponds to the certain measured projection group and is calculated by transforming the first computed tomography image in accordance with the transformation to be determined and by forward projecting the transformed first computed tomography image,

wherein the reconstruction unit reconstructs a motion corrected three-dimensional second computed tomography image based on the measured projections and the transformations determined for the different measured projection groups, wherein the computed tomography image generation apparatus comprises an examination zone in which the human head is arrangable, wherein the reconstruction unit reconstructs the first computed tomography image and the second computed tomography image such that they show the examination zone and to, for generating the second computed tomography image, perform the motion correction for a first part of the examination zone and to perform the motion correction not for a second part of the examination zone, wherein the first part of the examination zone includes the human head.

In another aspect of the present invention a computer program for controlling a computed tomography image generation apparatus as defined in claim 1 is presented, the computer program comprising program code means for causing the computed tomography image generation apparatus to carry out the steps of the computed tomography image generation method as defined in claim 14, when the computer program is run on a computer controlling the computed tomography image generation apparatus.

It shall be understood that the computed tomography image generation apparatus of claim 1, the computed tomography image generation method of claim 14, and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily an embodiment of a computed tomography image generation apparatus for generating an image of a human head,
Fig. 2 illustrates a first computed tomography image,
Fig. 3 illustrates a calculated projection obtained by forward projecting the first computed tomography image,
Fig. 4 illustrates a gradient angle difference image, and
Fig. 5 shows a flowchart exemplarily illustrating an embodiment of a computed tomography image generation method.

DETAILED DESCRIPTION OF EMBODIMENTS

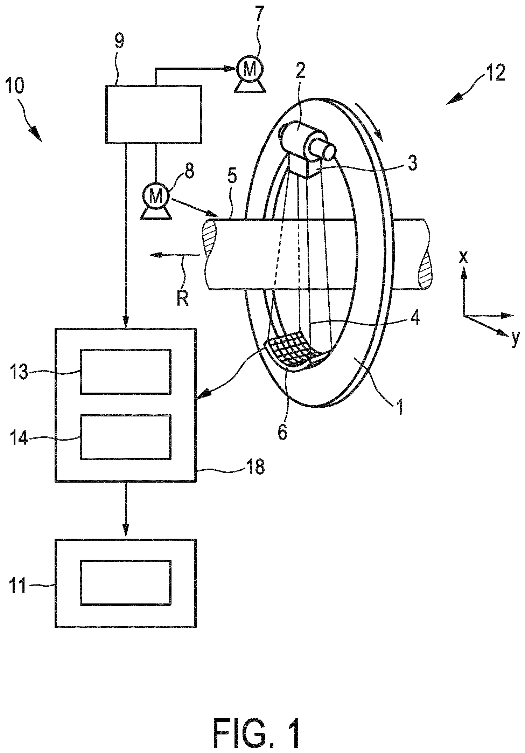

Fig. 1 shows schematically and exemplarily a computed tomography image generation apparatus 10 for generating an image of a human head. The computed tomography image generation apparatus 10 comprises a gantry 1 which is capable of rotation about a rotational axis R which extends parallel to the z direction. A radiation source 2, which is, in this embodiment, an x-ray tube, is mounted on the gantry 1. The radiation source 2 is provided with a collimator 3, which forms, in this embodiment, a conical radiation beam 4 from the radiation generated by the radiation source 2. The radiation traverses a human head of a patient (not shown) within an examination zone 5 being, in this embodiment, cylindrical. After having traversed the examination zone 5 and hence the human head, the radiation beam 4 is incident on a detection device 6 which comprises a two-dimensional detection surface. The detection device 6 is mounted on the gantry 1.

The computed tomography image generation apparatus 10 comprises two motors 7, 8. The gantry 1 is driven at a preferably constant but adjustable angular speed by the motor 7. The motor 8 is provided for displacing the patient arranged on a patient table in the examination zone 5 parallel to the direction of the rotational axis R or the z axis. These motors 7, 8 are controlled by a control unit 9, for instance, such that the radiation source 2 and the human head within the examination zone 5 move relative to each other along a helical trajectory. However, it is also possible that the radiation source 2 and the human head move relatively to each other along another trajectory. For instance, in an embodiment a step-and-shoot acquisition scheme can be used, wherein firstly the radiation source 2 is moved around the human head along a circular trajectory at a first longitudinal position of the radiation source 2 with respect to the patient, wherein then the patient table is moved such that the radiation source 2 is located at another longitudinal position with respect to the patient, wherein at this other longitudinal position the radiation source 2 is moved around the human head along a circular trajectory. The patient table can then be moved again, in order to acquire further projections by moving the radiation source 2 around the human head along a circular trajectory at another longitudinal position of the radiation source 2 with respect to the patient, and so forth.

During a relative movement of the radiation source 2 and the human head the detection device 6 generates measured values depending on the radiation incident on the detection surface of the detection device 6. Measured values, which have been generated at a same time while the radiation source 2 was at a same position relative to the human head, form a measured projection. The radiation source 2, the elements for moving the radiation source 2 relative to the human head, in particular, the motors 7, 8 and the gantry 1, and the detection device 6 can therefore be regarded as being components of a projections providing unit 12 being, in this embodiment, a projection acquisition device 12, for providing measured two-dimensional projections of the human head.

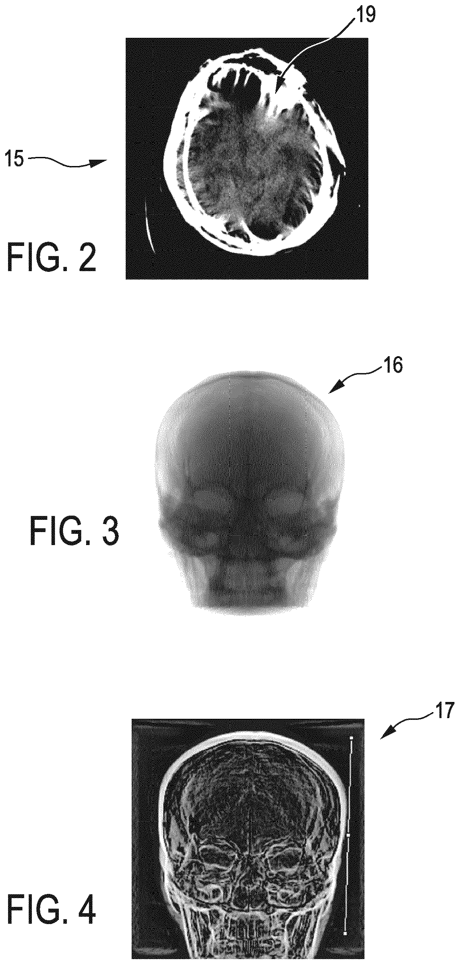

The measured projections are provided to a projection processing device 18 comprising a reconstruction unit 13 and a transformation determination unit 14, wherein also the projection processing device 18 is preferentially controlled by the control unit 9. The reconstruction unit 13 is adapted to reconstruct a three-dimensional first computed tomography image 15 of the human head based on the measured projections. The reconstruction unit 13 can be adapted to use known reconstruction algorithms like a filtered back projection algorithm or a Radon inversion algorithm for reconstructing the first computed tomography image. An example of a first computed tomography image 15 is illustrated in Fig. 2. As can be seen in Fig. 2, the reconstructed first computed tomography image comprises motion artifacts 19. The reconstruction unit 13 is preferentially adapted to reduce the motion artifacts 19 in the first computed tomography image 15 by using overscan weighting, which can also be regarded as redundant ray weighting. The minimum amount of projections required by the reconstruction unit 13 for reconstructing the first computed tomography image corresponds to an acquisition in which each voxel of the first computed tomography image to be reconstructed sees the radiation source 2 over an angular range of 180 degrees in a parallel beam geometry or over an angular range of 180 degrees plus fan angle in a focus centered geometry. Additional rays, i.e. additional projection values, can be used to apply a smooth weighing at the beginning and end of a respective 180 degrees segment or 180 degrees plus fan angle segment, respectively, wherein the weights can decrease within increasing distance from the mid of the respective segment. For more details regarding this overscan weighting reference is made to, for instance, pages 264 to 266 in the book "Computed Tomography Principles, Design, Artifacts, and Recent Advances" by Jiang Hsieh, second edition, John Wiley & Sons (2009), which are herewith incorporated by reference.

The transformation determination unit 14 is adapted to determine three-dimensional transformations of the first computed tomography image 15 of the head for different measured projection groups. Generally, a measured projection group can comprise a single measured projection only or several measured projections. In this embodiment each measured projection group comprises a single measured projection. The three-dimensional transformation determination unit 14 is adapted to determine for each measured projection group a transformation such that a degree of similarity between the respective measured projection group and a calculated projection group is increased, wherein the calculated projection group corresponds to the respective measured projection group and is calculated by transforming the first computed tomography image 15 in accordance with the transformation to be determined and by forward projecting the transformed first computed tomography image 15. That a measured projection group corresponds to a calculated projection group means that for the forward projection of the transformed first computed tomography image 15 the same acquisition geometry is used as used when acquiring the respective measured projection group. In this embodiment each calculated projection group comprises a single calculated projection only, wherein a calculated projection 16 is exemplarily shown in Fig. 3.

The transformation determination unit 14 is preferentially adapted to determine the degree of similarity by applying a similarity measure on the respective measured projection group and the respective corresponding calculated projection group, wherein this similarity measure is preferentially the normalized gradient angle difference as disclosed, for instance, in the article "Evaluation of optimization methods for intensity-based 2D-3D registration in x-ray guided interventions" by I.M.J. van der Bom et al., SPIE Medical Imaging (2011), which is herewith incorporated by reference. However, also other similarity measures can be used like a gradient correlation measure or a pattern intensity measure, which are also disclosed in the article by I.M.J. van der Bom, a sum of squared differences, et cetera. A gradient angle difference 17 is schematically and exemplarily illustrated in Fig. 4.

The transformation determination unit 14 is preferentially adapted to determine a transformation for a measured projection group iteratively. In particular, in each iteration step the transformation, which has been determined in the previous iteration step, is modified such that the degree of similarity between the respective measured projection group and the calculated projection group is increased, wherein these iteration steps may be performed until an abort criterion is fulfilled. The abort criterion is, for instance, that the degree of similarity between the respective measured projection group and the calculated projection group is larger than a predefined threshold or that a predefined maximum number of iterations has been reached.

The transformation determination unit 14 is preferentially further adapted to filter the determined transformations. In particular, the transformation determination unit 14 can be adapted to apply a smoothing filter like a median filter or another smoothing filter to the determined transformations. Moreover, the transformation determination unit 14 can be adapted to determine transformations based on the degree of similarity and the forward projection not for all times, at which projections have been measured, and to determine transformations for times, at which transformations have not been determined based on the degree of similarity and the forward projection, by interpolation. The interpolation can be a linear interpolation applied to corresponding parameters of the transformations. Higher orders of interpolation are of course also possible.

The transformations determination unit 14 can also be adapted to determine an outlier in the determined transformations and to remove the determined outlier from the determined transformations. The removed outlier transformation may be replaced by a transformation which has been determined by interpolation, wherein an interpolation algorithm may be applied to temporally neighboring transformations. The replacing transformation may also be determined in another way, for instance, by determining the median of the temporally neighboring transformations. For determining the replacing transformation one or several transformations corresponding to earlier times and one or several transformations corresponding to later times may be used.

The transformations determined for the different measured projection groups and hence for different times describe the motion of the head. The determined transformations can therefore be used by the reconstruction unit 13 to reconstruct a motion corrected three-dimensional second computed tomography image based on the measured projections. For reconstructing the motion corrected three-dimensional second computed tomography image known motion-correcting reconstruction algorithms may be used like the algorithms disclosed in the articles "Theoretical framework for a dynamic cone-beam reconstruction algorithm based on a dynamic particle model" by P. Grangeat et al.,

Physics in Medicine and Biology, volume 47, pages 2611 to 2625 (2002), "Compensation of Some Time Dependent Deformations in Tomography" by L. Desbat et al., IEEE Transactions on Medical Imaging, volume 26, number 2, pages 261 to 269 (2007), and "Dynamic X-Ray Computed Tomography" by S. Bonnet et al., Proceedings of the IEEE, volume 91, number 10, pages 1574 to 1587 (2003), which are herewith incorporated by reference. One exemplary way of reconstructing a motion correction three-dimensional second computed tomography image uses the inverse of the estimated motion, i.e. of the determined transformations, for compensating the motion as will be illustrated in the following.

If projections $p(\alpha,u,v)$, ramp filtered projections $rfp(\alpha,u,v)$ and a reconstructed image $f(x,y,z)$ are assumed, a filtered backprojection reconstruction can be formulated as

$$f(x,y,z) = \beta \sum_{\alpha} f(\alpha,x,y,z) \quad \text{with} \qquad (1)$$

$$f(\alpha,x,y,z) = BP \cdot w(\alpha,u,v) \cdot rfp(\alpha,u,v) \qquad , \qquad (2)$$

wherein $w(\alpha,u,v)$ is a known weight that considers redundant rays, $\beta$ is a scaling constant, $\alpha$ denotes a rotational angle of the radiation source 2 and $BP$ is the back projection operator connecting the image space with the projection domain. In case that $M$ is a transformation matrix, which preferentially includes translation and rotation and which is used to optimally fit the volume to a projection $p(\alpha,u,v)$, following can be defined:

$$MIf(x,y,z) = \beta \sum_{\alpha} MIf(\alpha,x,y,z) \qquad \text{with} \qquad (3)$$

$$MIf(\alpha,x,y,z) = MIBP \cdot w(\alpha,u,v,M) \cdot rfp(\alpha,u,v) \qquad . \qquad (4)$$

The weights w are adapted according to the transformation matrix M, because due to the motion a non-equiangular sampling may be generated. *MI* is the inverse motion operator in the image space which takes the result after back projecting a single view and compensates the estimated translation and rotation. The motion-corrected second computed tomography image is denoted by *MIf(x,y,z)*. The use of the inverse of the estimated motion, i.e. of the determined transformations, can of course also be formulated in another way.

The reconstructed second computed tomography image is provided to a display unit 11 for displaying the generated second computed tomography image. Also the first computed tomography image, the measured projections and/or the calculated projections may be shown on the display unit 11.

The reconstruction unit 13 and the transformation determination unit 14 can be adapted such that the second computed tomography image is iteratively reconstructed, wherein, after the second computed tomography image has been reconstructed, in an iteration step the transformations are determined again based on the second computed tomography image, which then has the function of the first computed tomography image, and the second computed tomography image is again reconstructed based on the newly determined transformations and the measured projections. The iteration can be performed, until an abort criterion is fulfilled. The abort criterion might be, for instance, that a difference between a current second computed tomography image and a previous second computed tomography image is smaller than a predefined threshold, or that a maximum number of iterations has been reached.

In an embodiment the reconstruction unit 13 can be adapted to divide the measured projections into several sets of measured projections, wherein each set includes temporally adjacent measured projections, and to reconstruct several three-dimensional first computed tomography images of the head based on the several sets of measured projections, wherein a respective first computed tomography image is reconstructed based on a respective set of measured projections. In this embodiment the reconstruction unit 13 is adapted to divide the measured projections into several sets of measured projections such that a respective set of measured projections does not fulfill the completeness criterion for computed tomography reconstruction. Thus, the reconstruction unit 13 is adapted to reconstruct each first computed tomography image by using an incomplete set of measured projections. Preferentially, the reconstruction unit 13 is adapted to perform this reconstruction of the first computed tomography images by using known tomosynthesis reconstruction algorithms, wherein the respective set of measured projections might be weighted by using a weighting function. Preferentially, the weighting function is adapted to provide a weighting factor which decreases towards the edges of the angular range to which the measured projections of the respective set correspond. The weighting function might be, for instance, a trapezoidal weighting function, a Gaussian weighting function or a Hanning weighting function.

The transformation determination unit 14 can be adapted to determine for each first computed tomography image a set of three-dimensional transformations of the respective first computed tomography image of the head for different measured projection groups, wherein a measured projection group comprises one or several measured projections of the respective set of measured projections. Moreover, the transformation determination unit 14 can be adapted to determine for a respective measured projection group of the respective set of measured projections a transformation such that a degree of similarity between the respective measured projection group and a calculated projection group is increased, wherein the calculated projection group corresponds to the respective measured projection group and is calculated by transforming the respective first computed tomography image in accordance with the transformation to be determined and by forward projecting the transformed respective first computed tomography image.

The transformation determination unit 14 can be further adapted to determine further transformations transforming pairs of transformed first computed tomography images, which have been reconstructed based on neighboring sets of measured projections and which have then be transformed by using neighboring measured projection groups which belong to the neighboring sets of measured projections, i.e. one of these measured projection groups belongs to one of these sets of projections and the other of these measured projection groups belongs to the other of the sets of measured projections. Moreover, the reconstruction unit 13 can be adapted to reconstruct the motion corrected three-dimensional second computed tomography image based on the several sets of transformations determined for the different measured projections groups of the several sets of measured projections, the further transformations transforming first computed tomography images of different sets of measured projections such that they correspond to each and the measured projections.

For instance, if the radiation source 2 is rotated around the head along a circular or helical trajectory over 360 degrees while measuring the projections, the reconstruction unit 13 may divide the angular range of 360 degrees into angular sub ranges of 20 degrees. Thus, a first set of measured projections may correspond to an angular sub range from 0 to 20 degrees, a second set of measured projections may correspond to an angular sub range from 21 to 40 degrees, a third set of measured projections may correspond to an angular sub range from 41 to 60 degrees, et cetera. The reconstructing 13 can be adapted to reconstruct for each of these sets of measured projections, i.e. for each of these angular ranges, a respective first computed tomography image. The transformation unit 14 may then be adapted to determine for each of these sets of measured projections, i.e. for each of these angular ranges, transformations by using measured projection groups of the respective set of measured projection groups. For example, in order to determine the transformations for the first angular range, a first measured projection group corresponding to angular positions at 1 and 2 degrees, a second measured projection group corresponding to angular positions at 3 and 4 degrees, a third measured projection group corresponding to angular positions at 5 and 6 degrees, et cetera can be used. For each of these angular sub-sub ranges, i.e. a) 1 and 2 degrees, b) 3 and 4 degrees, c) 5 and 6 degrees, et cetera, a respective transformation can be determined, in order to determine for the angular sub range from 0 to 20 degrees a first set of transformations. A corresponding set of transformations can be determined for each of the other angular sub ranges, i.e. for the second angular sub range from 20 to 40 degrees, for the third angular sub range from 40 to 60 degrees, et cetera. Further transformations can be determined by registering the transformed first computed tomography image, which has been determined for the angular positions at 19 and 20 degrees for the first set of measured projections, with the transformed first computed tomography image which has been determined for the angular positions at 21 and 22 degrees for the second set of measured projections. Correspondingly, a further transformation can be determined by registering the transformed reconstructed first computed tomography images determined for the angular positions 39 and 40 degrees and 41 and 42 degrees, which belong to the second and third sets of measured projections, such that these transformed first computed tomography images correspond to each other. This determination of further transformations can be continued with further neighboring transformed first computed tomography images at borders of neighboring sets of measured projections, in order to determine for the entire angular range of 360 degrees the further transformations. The reconstruction unit 13 can then be adapted to use all measured projections covering the 360 degrees together with the sets of transformations determined for the different sets of measured projections and the further transformations for reconstructing the motion-corrected second computed tomography image.

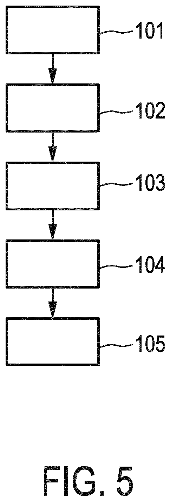

In the following an embodiment of a computed tomography image generation method for generating an image of a human head will exemplarily be described with reference to a flowchart shown in Fig. 5.

In step 101 the radiation source 2 rotates around the rotational axis R and the patient, especially the human head to be imaged, is moved along the rotational axis R such that the radiation source 2 moves relative to the human head along a helical trajectory. In another embodiment the radiation source 2 and the human head can be moved relatively to each other in another way, for instance, in a step-and-shoot acquisition scheme. The radiation source 2 emits radiation traversing the human head and the detection device 6 detects the radiation, which has traversed the human head, and generates measured projections based on the detected radiation.

The measured projections are transferred to the projection processing device 18 and in step 102 the registration unit 13 reconstructs a three-dimensional first computed tomography image of the head based on the measured projections. In step 103 the transformation determination unit 14 determines three-dimensional transformations of the first computed tomography image of the head for different measured projection groups, wherein in this embodiment each measured projection group comprises a single measured projection. The transformation determination unit 14 determines for a certain measured projection group a transformation such that a degree of similarity between the certain measured projection group and a calculated projection group is increased, especially optimized, wherein the calculated projection group corresponds to the certain measured projection group and is determined by transforming the first computed tomography image in accordance with the transformation to be determined and by forward projecting the transformed first computed tomography image.

In step 104 the reconstruction unit 13 reconstructs a motion corrected three-dimensional second computed tomography image based on the measured projections and the transformations determined for the different measured projection groups, wherein in step 105 the reconstructed motion corrected three-dimensional second computed tomography image is shown on the display unit 11.

The image generation apparatus and the image generation method use a second path motion estimation and compensation scheme to reduce motion artifacts during brain computed tomography scans, i.e. in reconstructed computed tomography images of a human head. As a prerequisite a first three-dimensional representation of the head is required, which is reconstructed from the available measured projections. Motion artifacts may be partly reduced in this first three-dimensional representation, i.e. in the first computed tomography image, by using known motion reduction techniques like overscan weighting techniques. The three-dimensional representation of the human head is then forward projected for generating calculated projections, which are compared with the measured projections, in order to estimate the three-dimensional motion of the human head, wherein the estimated three-dimensional motion is subsequently used in a motion compensated reconstruction scheme.

Thus, a three-dimensional head volume data set is reconstructed from the measured projections, wherein the measured projections may be acquired in a helical or step-and-shoot acquisition scheme. For each measured projection or for an angular subset of the measured projections, i.e. for each measured projection group, a forward projection of the reconstructed volume is preferentially calculated, in order to generate a calculated projection group which is compared to the respective measured projection group using a similarity measure being, for instance, a gradient angle difference, which may be normalized. In an embodiment the transformation determination unit, which can be regarded as being an optimizer, then determines the best three-dimensional transformation for the respective measured projection group to align the volume with a single measured projection or with multiple measured projections with a small angular distance. For instance, a respective measured projection group may comprise several measured projections which correspond to an angular range of, for instance, 20 degrees or less, 10 degrees or less, or 5 degrees or less. The resulting transformation, which is determined for the respective measured projection group, is stored as a motion representation for this measured projection group. After the transformations have been determined for the different measured projection groups, an angular array of transformations is present, i.e. transformations determined for different measured projection groups and hence for different times is present, wherein these transformations may be filtered, interpolated or outliers may be reduced. A motion compensated reconstruction may then be used to reconstruct an improved volume, wherein preferentially the inverse of the estimated motion is used to compensate the motion. The method may be applied as an iterative method.

In an embodiment the transformation determination unit is adapted to determine whether the first computed tomography image comprises motion artifacts or not. If it is determined that the first computed tomography image does not comprise motion artifacts, the three-dimensional transformations are not determined and correspondingly a motion corrected three-dimensional second computed tomography image is not reconstructed. For determining whether the first computed tomography image comprises motion artifacts or not, known motion artifacts detection algorithms can be used. For instance, the transformation determination unit can be adapted to detect streaks originating from edges in the first computed tomography image or other artifacts which are known to be caused by motion. For detecting these artifacts in the first computed tomography image known segmentation algorithms can be used. The reconstruction unit can also be adapted to compare redundant measured projections, which correspond to opposing acquisition directions, after they have been rebinned into parallel ray geometry, in order to determine whether motion has caused artifacts in the first computed tomography image. If a difference between these parallel rebinned projections, which correspond to opposite acquisition directions, is smaller than a predefined threshold, it can be assumed that no motion is present.

In an embodiment the reconstruction unit is adapted to, for generating the second image computed tomography image, perform the motion correction for a first part of the examination zone and to perform the motion correction not for a second part of the examination zone. The first part preferentially includes the human head, whereas the second part may include the patient table.

Although in above described embodiments each measured projection group and each calculated projection group comprises a single projection only, in other embodiments some or all measured projection groups and calculated projection groups may comprise several projections. In an embodiment all measured projection groups and all calculated projection groups comprise a same number of projections. However, in another embodiment, the number of projections can be different for different measured projection groups and different calculated projection groups.

Although in an above described embodiment a certain computed tomography system with a rotatable gantry and a radiation source and a detection device mounted to the gantry has been described, in other embodiments the computed tomography image generation apparatus can be another apparatus for generating a computed tomography image. For instance, it can be a C-arm computed tomography system which might have a flat panel detector. Moreover, the projections providing unit may just be a storing unit which can provide stored, already measured projections or a receiving unit for receiving the measured projections and for providing the received projections such that the computed tomography image generation apparatus can be a computing device being adapted to generate a computed tomography image of the head.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or devices may fulfill the functions of several items recited in the claims. The mere fact that certain measures are recited in mutually different dependent claims does not indicate that a combination of these measures cannot be used to advantage.

Procedures like the reconstruction of the first computed tomography image, the determination of the transformations, in particular, the forward projection procedures and the determination of the degree of similarity, the reconstruction of the second computed tomography image, et cetera performed by one or several units or devices can be performed by any other number of units or devices. For example, steps 102 to 104 can be performed by a single unit or by any other number of different units. The procedures and/or the control of the image generation apparatus in accordance with the image generation method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a CT image generation apparatus for generating an image of a head. Transformations of a first CT image of the head are determined for different measured projection groups, wherein for a measured projection group a transformation is determined such that a degree of similarity between the measured projection group and a calculated projection group is increased, wherein the calculated projection group is calculated by transforming the first CT image in accordance with the transformation to be determined and by forward projecting the transformed first CT image. A motion corrected three-dimensional second CT image is reconstructed based on the measured projections and the transformations determined for the different measured projection groups. This allows providing a high-quality CT image of the head, even if a patient cannot stop moving the head in case of, for instance, stroke.

**Claims**

1. A computed tomography image generation apparatus for generating an image of a human head, wherein the computed tomography image generation apparatus (10) comprises:

   - a projections providing unit (12) for providing measured two-dimensional projections of the head, wherein the measured projections have been measured at different times while a radiation source (2), which emits radiation (4) for traversing the head, has been moved around the head and wherein the measured projections have been generated based on the radiation (4) after having traversed the head,
   - a reconstruction unit (13) for reconstructing a three-dimensional first computed tomography image (15) of the head based on the provided measured projections,
   - a transformation determination unit (14) for determining three-dimensional transformations of the first computed tomography image (15) of the head for different measured projection groups, wherein a measured projection group comprises one or several measured projections, wherein the transformation determination unit (14) is adapted to determine for a certain measured projection group a transformation such that a degree of similarity between the certain measured projection group and a calculated projection group (16) is increased, wherein the calculated projection group (16) corresponds to the certain measured projection group and is calculated by transforming the first computed tomography image (15) in accordance with the transformation to be determined and by forward projecting the transformed first computed tomography image (15),

   wherein the reconstruction unit (13) is adapted to reconstruct a motion corrected three-dimensional second computed tomography image based on the measured projections and the transformations determined for the different measured projection groups,

wherein the computed tomography image generation apparatus (10) comprises an examination zone in which the human head is arrangeable,

wherein the reconstruction unit (13) is adapted to reconstruct the first computed tomography image and the second computed tomography image such that they show the examination zone and to, for generating the second computed tomography image, perform the motion correction for a first part of the examination zone and to perform the motion correction not for a second part of the examination zone, wherein the first part of the examination zone includes the human head.

2. The computed tomography image generation apparatus as defined in claim 1, wherein the transformation determination unit (14) is adapted to use a gradient angle difference for determining the degree of similarity.

3. The computed tomography image generation apparatus as defined in claim 1, wherein the reconstruction unit (13) is adapted to reduce motion artifacts in the first computed tomography image (15) by using overscan weighting.

4. The computed tomography image generation apparatus as defined in claim 1, wherein the transformation determination unit (14) is adapted to determine a transformation for a measured projection group iteratively.

5. The computed tomography image generation apparatus as defined in claim 1, wherein the transformation determination unit (14) is adapted such that each measured projection group comprises a single measured projection.

6. The computed tomography image generation apparatus as defined in claim 1, wherein the transformation determination unit (14) is adapted such that each measured projection group comprises several measured projections.

7. The computed tomography image generation apparatus as defined in claim 1, wherein the transformation determination unit (14) is adapted to filter the determined transformations.

8. The computed tomography image generation apparatus as defined in claim 1, wherein the transformation determination unit (14) is adapted to determine transformations based on the degree of similarity and the forward projection not for all times, at which projections have been measured, and to determine transformations for times, at which transformations have not been determined based on the degree of similarity and the forward projection, by interpolation.

9. The computed tomography image generation apparatus as defined in claim 1, wherein the transformation determination unit (14) is adapted to determine an outlier in the determined transformations and to remove the determined outlier from the determined transformations.

10. The computed tomography image generation apparatus as defined in claim 1, wherein:

    - the reconstruction unit (13) is adapted to divide the measured projections into several sets of measured projections, wherein each set includes temporally adjacent measured projections, and to reconstruct several three-dimensional first computed tomography images of the head based on the several sets of measured projections, wherein a respective first computed tomography image is reconstructed based on a respective set of measured projections,

    - the transformation determination unit (14) is adapted to determine for each first computed tomography image a set of three-dimensional transformations of the respective first computed tomography image of the head for different measured projection groups, wherein a measured projection group comprises one or several measured projections of the respective set of measured projections, wherein the transformation determination unit (14) is adapted to determine for a certain measured projection group of the respective set of measured projections a transformation such that a degree of similarity between the certain measured projection group and a calculated projection group is increased, wherein the calculated projection group corresponds to the certain measured projection group and is calculated by transforming the respective first computed tomography image in accordance with the transformation to be determined and by forward projecting the transformed respective first computed tomography image,

    - the reconstruction unit (13) is adapted to reconstruct a motion corrected three-dimensional second computed tomography image based on the measured projections and the several sets of transformations determined for the different measured projection groups of the several sets of measured projections.

11. The computed tomography image generation apparatus as defined in claim 10, wherein the transformation determination unit (14) is adapted to determine a further transformation transforming a transformed first computed tomography image, which has been reconstructed based on a set of measured projections and which has then be transformed, such that it corresponds to another transformed first computed tomography image, which has been reconstructed based on another set of measured projections and which has then be transformed, wherein the reconstruction unit (13) is adapted to reconstruct the motion corrected three-dimensional second computed tomography image also based on the further transformation.

12. The computed tomography image generation apparatus as defined in claim 10, wherein the reconstruction unit (13) is adapted to divide the measured projections into several sets of measured projections such that at least one set of measured projections does not fulfill the completeness criterion for computed tomography reconstruction.

13. The computed tomography image generation apparatus as defined in claim 1, wherein the reconstruction unit (13) and the transformation determination unit (14) are adapted such that, after the second computed tomography image has been reconstructed, in an iteration step the transformations are determined again based on the second computed tomography image, which then has the function of the first computed tomography image, and the second computed tomography image is again reconstructed based on the newly determined transformations and the measured projections.

14. A computed tomography image generation method for generating an image of a human head, wherein the computed tomography image generation method comprises:

- providing measured two-dimensional projections of the head by a projections providing unit (12), wherein the measured projections have been measured at different times while a radiation source (2), which emits radiation (4) for traversing the head, has been moved around the head and wherein the measured projections have been generated based on the radiation (4) after having traversed the head,
- reconstructing a three-dimensional first computed tomography image (15) of the head based on the provided measured projections by a reconstruction unit (13),
- determining three-dimensional transformations of the first computed tomography image of the head for different measured projection groups by a transformation determination unit (14), wherein a measured projection group comprises one or several measured projections, wherein the transformation determination unit (14) determines for a certain measured projection group a transformation such that a degree of similarity between the certain measured projection group and a calculated projection group (16) is increased, wherein the calculated projection group (16) corresponds to the certain measured projection group and is calculated by transforming the first computed tomography image (15) in accordance with the transformation to be determined and by forward projecting the transformed first computed tomography image (15),

wherein the reconstruction unit (13) reconstructs a motion corrected three-dimensional second computed tomography image based on the measured projections and the transformations determined for the different measured projection groups, wherein the computed tomography image generation apparatus (10) comprises an examination zone in which the human head is arrangeable, wherein the reconstruction unit (13) reconstructs the first computed tomography image and the second computed tomography image such that they show the examination zone and to, for generating the second computed tomography image, perform the motion correction for a first part of the examination zone and to perform the motion correction not for a second part of the examination zone, wherein the first part of the examination zone includes the human head.

15. A computer program for controlling a computed tomography image generation apparatus as defined in claim 1, the computer program comprising program code means for causing the computed tomography image generation apparatus to carry out the steps of the computed tomography image generation method as defined in claim 14, when the computer program is run on a computer controlling the computed tomography image generation apparatus.


**Patentansprüche**

1. Computertomografiebilderzeugungsvorrichtung zum Erzeugen eines Bilds eines menschlichen Kopfs, wobei die Computertomografiebilderzeugungsvorrichtung (10) Folgendes umfasst:

- eine Projektionsbereitstellungseinheit (12) zum Bereitstellen gemessener zweidimensionaler Projektionen des Kopfs, wobei die gemessenen Projektionen zu verschiedenen Zeitpunkten gemessen wurden, während sich eine Strahlungsquelle (2), die Strahlung (4) zum Durchqueren des Kopfs emittiert, um den Kopf herum bewegte, und wobei die gemessenen Projektionen basierend auf der Strahlung (4) nach Durchqueren des Kopfs erzeugt wurden,

- eine Rekonstruktionseinheit (13) zum Rekonstruieren eines dreidimensionalen ersten Computertomografiebilds (15) des Kopfs basierend auf den bereitgestellten gemessenen Projektionen,

- eine Transformationsbestimmungseinheit (14) zum Bestimmen von dreidimensionalen Transformationen des ersten Computertomografiebilds (15) des Kopfs für verschiedene gemessene Projektionsgruppen, wobei eine gemessene Projektionsgruppe eine oder mehrere gemessene Projektionen umfasst, wobei die Transformationsbestimmungseinheit (14) dafür ausgelegt ist, für eine gewisse gemessene Projektionsgruppe eine Transformation derartig zu bestimmen, dass ein Ähnlichkeitsgrad zwischen der gewissen gemessenen Projektionsgruppe und einer berechneten Projektionsgruppe (16) erhöht wird, wobei die berechnete Projektionsgruppe (16) der gewissen gemessenen Projektionsgruppe entspricht und berechnet wird, indem das erste Computertomografiebild (15) in Übereinstimmung mit der zu bestimmenden Transformation transformiert wird und indem das transformierte erste Computertomografiebild (15) vorwärtsprojiziert wird,

wobei die Rekonstruktionseinheit (13) dafür ausgelegt ist, ein bezüglich der Bewegung korrigiertes dreidimensionales zweiten Computertomografiebild basierend auf den gemessenen Projektionen und den für die verschiedenen gemessenen Projektionsgruppen bestimmten Transformationen zu rekonstruieren,

wobei die Computertomografiebilderzeugungsvorrichtung (10) eine Untersuchungszone umfasst, in der der menschliche Kopf angeordnet werden kann, wobei die Rekonstruktionseinheit (13) dafür ausgelegt ist, das erste Computertomografiebild und das zweite Computertomografiebild derartig zu rekonstruieren, dass sie die Untersuchungszone zeigen, und, zum Erzeugen des zweiten Computertomografiebilds, die Korrektur bezüglich der Bewegung für einen ersten Teil der Untersuchungszone durchzuführen und die Korrektur bezüglich der Bewegung nicht für einen zweiten Teil der Untersuchungszone durchzuführen, wobei der erste Teil der Untersuchungszone den menschlichen Kopf einschließt.

2. Computertomografiebilderzeugungsvorrichtung nach Anspruch 1, wobei die Transformationsbestimmungseinheit (14) dafür ausgelegt ist, eine Gradientenwinkeldifferenz zum Bestimmen des Ähnlichkeitsgrads zu verwenden.

3. Computertomografiebilderzeugungsvorrichtung nach Anspruch 1, wobei die Rekonstruktionseinheit (13) dafür ausgelegt ist, Bewegungsartefakte in dem ersten Computertomografiebild (15) durch Verwendung von Overscan-Gewichtung zu reduzieren.

4. Computertomografiebilderzeugungsvorrichtung nach Anspruch 1, wobei die Transformationsbestimmungseinheit (14) dafür ausgelegt ist, eine Transformation für eine gemessene Projektionsgruppe iterativ zu bestimmen.

5. Computertomografiebilderzeugungsvorrichtung nach Anspruch 1, wobei die Transformationsbestimmungseinheit (14) derartig ausgelegt ist, dass jede gemessene Projektionsgruppe eine einzelne gemessene Projektion umfasst.

6. Computertomografiebilderzeugungsvorrichtung nach Anspruch 1, wobei die Transformationsbestimmungseinheit (14) derartig ausgelegt ist, dass jede gemessene Projektionsgruppe mehrere gemessene Projektionen umfasst.

7. Computertomografiebilderzeugungsvorrichtung nach Anspruch 1, wobei die Transformationsbestimmungseinheit (14) dafür ausgelegt ist, die bestimmten Transformationen zu filtern.

8. Computertomografiebilderzeugungsvorrichtung nach Anspruch 1, wobei die Transformationsbestimmungseinheit (14) dafür ausgelegt ist, Transformationen basierend auf dem Ähnlichkeitsgrad und der Vorwärtsprojektion nicht für alle Zeitpunkte zu bestimmen, an denen Projektionen gemessen wurden, und Transformationen für Zeitpunkte, an denen Transformationen nicht basierend auf dem Ähnlichkeitsgrad und der Vorwärtsproj ektion bestimmt wurden, durch Interpolation zu bestimmen.

9. Computertomografiebilderzeugungsvorrichtung nach Anspruch 1, wobei die Transformationsbestimmungseinheit (14) dafür ausgelegt ist, einen Ausreißer in den bestimmten Transformationen zu bestimmen und den bestimmten Ausreißer aus den bestimmten Transformationen zu entfernen.

10. Computertomografiebilderzeugungsvorrichtung nach Anspruch 1, wobei:

- die Rekonstruktionseinheit (13) dafür ausgelegt ist, die gemessenen Projektionen in mehrere Sätze von gemessenen Projektionen zu unterteilen, wobei jeder Satz zeitlich benachbarte gemessene Projektionen einschließt, und mehrere dreidimensionale erste Computertomografiebilder des Kopfs basierend auf den mehreren Sätzen von gemessenen Projektionen zu rekonstruieren, wobei ein jeweiliges erstes Computertomografiebild basierend auf einem jeweiligen Satz von gemessenen Projektionen rekonstruiert wird,

- die Transformationsbestimmungseinheit (14) dafür ausgelegt ist, für jedes erste Computertomografiebild einen Satz von dreidimensionalen Transformationen des jeweiligen ersten Computertomografiebilds des Kopfs für verschiedene gemessene Projektionsgruppen zu bestimmen, wobei eine gemessene Projektionsgruppe eine oder mehrere gemessene Projektionen des jeweiligen Satzes von gemessenen Projektionen umfasst, wobei die Transformationsbestimmungseinheit (14) dafür ausgelegt ist, für eine gewisse gemessene Projektionsgruppe des jeweiligen Satzes von gemessenen Projektionen eine Transformation derartig zu bestimmen, dass ein Ähnlichkeitsgrad zwischen der gewissen gemessenen Projektionsgruppe und einer berechneten Projektionsgruppe erhöht wird, wobei die berechnete Projektionsgruppe der gewissen gemessenen Projektionsgruppe entspricht und berechnet wird, indem das jeweilige erste Computertomografiebild in Übereinstimmung mit der zu bestimmenden Transformation transformiert wird und indem das transformierte jeweilige erste Computertomografiebild vorwärtsprojiziert wird,

- die Rekonstruktionseinheit (13) dafür ausgelegt ist, ein bezüglich der Bewegung korrigiertes dreidimensionales zweites Computertomografiebild basierend auf den gemessenen Projektionen und den mehreren Sätzen von Transformationen zu rekonstruieren, für die verschiedenen gemessenen Projektionsgruppen der mehreren Sätze von gemessenen Projektionen bestimmt wurden.

11. Computertomografiebilderzeugungsvorrichtung nach Anspruch 10, wobei die Transformationsbestimmungseinheit (14) dafür ausgelegt ist, eine weitere Transformation zu bestimmen, die ein transformiertes erstes Computertomografiebild transformiert, das basierend auf einem Satz von gemessenen Projektionen rekonstruiert wurde und das dann transformiert wurde, sodass es einem weiteren transformierten ersten Computertomografiebild entspricht, das basierend auf einem weiteren Satz von gemessenen Projektionen rekonstruiert wurde und das dann transformiert wurde, wobei die Rekonstruktionseinheit (13) dafür ausgelegt ist, das bezüglich der Bewegung korrigierte dreidimensionale zweite Computertomografiebild auch basierend auf der weiteren Transformation zu rekonstruieren.

12. Computertomografiebilderzeugungsvorrichtung nach Anspruch 10, wobei die Rekonstruktionseinheit (13) dafür ausgelegt ist, die gemessenen Projektionen in mehrere Sätze von gemessenen Projektionen zu unterteilen, sodass mindestens ein Satz von gemessenen Projektionen das Vollständigkeitskriterium für Computertomografie-Rekonstruktion nicht erfüllt.

13. Computertomografiebilderzeugungsvorrichtung nach Anspruch 1, wobei die Rekonstruktionseinheit (13) und die Transformationsbestimmungseinheit (14) derartig ausgelegt sind, dass, nachdem das zweite Computertomografiebild rekonstruiert wurde, in einem Iterationsschritt die Transformationen erneut basierend auf dem zweiten Computertomografiebild bestimmt werden, welches dann die Funktion des ersten Computertomografiebilds hat, und das zweite Computertomografiebild erneut basierend auf den neu bestimmten Transformationen und den gemessenen Projektionen rekonstruiert wird.

14. Computertomografiebilderzeugungsverfahren zum Erzeugen eines Bilds eines menschlichen Kopfs, wobei das Computertomografiebilderzeugungsverfahren Folgendes umfasst:

- Bereitstellen gemessener zweidimensionaler Projektionen des Kopfs durch eine Projektionsbereitstellungseinheit (12), wobei die gemessenen Projektionen zu verschiedenen Zeitpunkten gemessen wurden, während sich eine Strahlungsquelle (2), die Strahlung (4) zum Durchqueren des Kopfs emittiert, um den Kopf herum bewegte, und wobei die gemessenen Projektionen basierend auf der Strahlung (4) nach Durchqueren des Kopfs erzeugt wurden,

- Rekonstruieren eines dreidimensionalen ersten Computertomografiebilds (15) des Kopfs basierend auf den bereitgestellten gemessenen Projektionen durch eine Rekonstruktionseinheit (13),

- Bestimmen von dreidimensionalen Transformationen des ersten Computertomografiebilds des Kopfs für verschiedene gemessene Projektionsgruppen durch eine Transformationsbestimmungseinheit (14), wobei eine gemessene Projektionsgruppe eine oder mehrere gemessene Projektionen umfasst, wobei die Transformationsbestimmungseinheit (14) für eine gewisse gemessene Projektionsgruppe eine Transformation derartig bestimmt, dass ein Ähnlichkeitsgrad zwischen der gewissen gemessenen Projektionsgruppe und einer berechneten Projektionsgruppe (16) erhöht wird, wobei die berechnete Projektionsgruppe (16) der gewissen gemessenen Projektionsgruppe entspricht und berechnet wird, indem das erste Computertomografiebild (15) in Übereinstimmung mit der zu bestimmenden Transformation transformiert wird und indem das transformierte erste Computertomografiebild (15) vorwärtsprojiziert wird,

wobei die Rekonstruktionseinheit (13) ein bezüglich der Bewegung korrigiertes dreidimensionales zweiten Computertomografiebild basierend auf den gemessenen Projektionen und den für die verschiedenen gemessenen Projektionsgruppen bestimmten Transformationen rekonstruiert, wobei die Computertomografiebilderzeugungsvorrichtung (10) eine Untersuchungszone umfasst, in der der menschliche Kopf angeordnet werden kann, wobei die Rekonstruktionseinheit (13) das erste Computertomografiebild und das zweite Computertomografiebild derartig rekonstruiert, dass sie die Untersuchungszone zeigen, und, zum Erzeugen des zweiten Computertomografiebilds, die Korrektur bezüglich der Bewegung für einen ersten Teil der Untersuchungszone durchführt und die Korrektur bezüglich der Bewegung nicht für einen zweiten Teil der Untersuchungszone durchführt, wobei der erste Teil der Untersuchungszone den menschlichen Kopf einschließt.

15. Computerprogramm zum Steuern einer Computertomografiebilderzeugungsvorrichtung nach Anspruch 1, wobei das Computerprogramm Programmcodemittel umfasst, um die Computertomografiebilderzeugungsvorrichtung zu veranlassen, die Schritte des Computertomografiebilderzeugungsverfahrens nach Anspruch 14 durchzuführen, wenn das Computerprogramm auf einem Computer ausgeführt wird, der die Computertomografiebilderzeugungsvorrichtung steuert.

**Revendications**

1. Appareil de production d'image de tomodensitométrie pour produire une image d'une tête humaine, dans lequel l'appareil de production d'image de tomodensitométrie (10) comprend :

   - une unité de fourniture de projections (12) pour fournir des projections mesurées bidimensionnelles de la tête, dans lesquelles les projections mesurées ont été mesurées à différents instants tandis qu'une source de rayonnement (2), qui émet un rayonnement (4) pour traverser la tête, a été déplacée autour de la tête et dans lequel les projections mesurées ont été produites sur la base du rayonnement (4) après avoir traversé la tête,
   - une unité de reconstruction (13) pour reconstruire une première image de tomodensitométrie tridimensionnelle (15) de la tête sur la base des projections mesurées fournies,
   - une unité de détermination de transformation (14) pour déterminer des transformations tridimensionnelles de la première image de tomodensitométrie (15) de la tête pour des groupes de projections mesurées différents, dans lesquels un groupe de projections mesurées comprend une ou plusieurs projections mesurées, dans laquelle l'unité de détermination de transformation (14) est conçue pour déterminer pour un certain groupe de projections mesurées une transformation de sorte qu'un degré de similitude entre le certain groupe de projections mesurées et un groupe de projections calculées (16) est augmenté, dans lequel le groupe de projections calculées (16) correspond au certain groupe de projections mesurées et est calculé en transformant la première image de tomodensitométrie (15) selon la transformation à déterminer et par la projection en avant de la première image de tomodensitométrie transformée (15),

   dans lequel l'unité de reconstruction (13) est conçue pour reconstruire une seconde image de tomodensitométrie tridimensionnelle à mouvement corrigé sur la base des projections mesurées et des transformations déterminées pour les différents groupes de projections mesurées,
   dans lequel l'appareil de production d'image de tomodensitométrie (10) comprend une zone d'examen dans laquelle la tête humaine peut être agencée,
   dans lequel l'unité de reconstruction (13) est conçue pour reconstruire la première image de tomodensitométrie et la seconde image de tomodensitométrie de sorte qu'elles montrent la zone d'examen et afin de, pour produire la seconde image de tomodensitométrie, effectuer la correction de mouvement pour une première partie de la zone d'examen et ne pas effectuer la correction de mouvement pour une seconde partie de la zone d'examen, dans lesquelles la première partie de la zone d'examen inclut la tête humaine.

2. Appareil de production d'image de tomodensitométrie comme défini dans la revendication 1, dans lequel l'unité de détermination de transformation (14) est conçue pour utiliser une différence angulaire de gradient pour déterminer le degré de similitude.

3. Appareil de production d'image de tomodensitométrie comme défini dans la revendication 1, dans lequel l'unité de reconstruction (13) est conçue pour réduire des artefacts de mouvement dans la première image de tomodensitométrie (15) en utilisant une pondération de surbalayage.

4. Appareil de production d'image de tomodensitométrie comme défini dans la revendication 1, dans lequel l'unité de détermination de transformation (14) est conçue pour déterminer de manière itérative une transformation pour un groupe de projections mesurées.

5. Appareil de production d'image de tomodensitométrie comme défini dans la revendication 1, dans lequel l'unité de détermination de transformation (14) est conçue de sorte que chaque groupe de projections mesurées comprend une projection mesurée unique.

6. Appareil de production d'image de tomodensitométrie comme défini dans la revendication 1, dans lequel l'unité de détermination de transformation (14) est conçue de sorte que chaque groupe de projections mesurées comprend plusieurs projections mesurées.

7. Appareil de production d'image de tomodensitométrie comme défini dans la revendication 1, dans lequel l'unité de détermination de transformation (14) est conçue pour filtrer les transformations déterminées.

8. Appareil de production d'image de tomodensitométrie comme défini dans la revendication 1, dans lequel l'unité de détermination de transformation (14) est conçue pour déterminer des transformations sur la base du degré de similitude et de la projection en avant pas pour tous les instants, auxquels les projections ont été mesurées, et pour déterminer des transformations pour des instants, auxquels des transformations n'ont pas été déterminées sur la base du degré de similitude et de la projection en avant, par interpolation.

9. Appareil de production d'image de tomodensitométrie comme défini dans la revendication 1, dans lequel l'unité de détermination de transformation (14) est conçue pour déterminer un point aberrant dans les transformations déterminées et pour enlever le point aberrant déterminé des transformations déterminées.

10. Appareil de production d'image de tomodensitométrie comme défini dans la revendication 1, dans lequel :

l'unité de reconstruction (13) est conçue pour diviser les projections mesurées en plusieurs ensembles de projections mesurées, dans lesquels chaque ensemble inclut des projections mesurées temporellement adjacentes, et pour reconstruire plusieurs premières images de tomodensitométrie tridimensionnelles de la tête sur la base de plusieurs ensembles de projections mesurées, dans lesquelles une première image de tomodensitométrie respective est reconstruite sur la base d'un ensemble respectif de projections mesurées,

l'unité de détermination de transformation (14) est conçue pour déterminer pour chaque première image de tomodensitométrie un ensemble de transformations tridimensionnelles de la première image de tomodensitométrie respective de la tête pour des groupes de projections mesurées différents, dans lesquels un groupe de projections mesurées comprend une ou plusieurs projections mesurées de l'ensemble respectif de projections mesurées, dans lequel l'unité de détermination de transformation (14) est conçue pour déterminer pour un certain groupe de projections mesurées de l'ensemble respectif de projections mesurées une transformation de sorte qu'un degré de similitude entre le certain groupe de projections mesurées et un groupe de projections calculées est augmenté, dans lequel le groupe de projections calculées correspond au certain groupe de projections mesurées et est calculé en transformant la première image de tomodensitométrie respective selon la transformation à déterminer et par projection en avant de la première image de tomodensitométrie respective transformée,

l'unité de reconstruction (13) est conçue pour reconstruire une seconde image de tomodensitométrie tridimensionnelle à mouvement corrigé sur la base des projections mesurées et de plusieurs ensembles de transformations déterminées pour les groupes de projections mesurées différents des plusieurs ensembles de projections mesurées.

11. Appareil de production d'image de tomodensitométrie comme défini dans la revendication 10, dans lequel l'unité de détermination de transformation (14) est conçue pour déterminer une transformation supplémentaire transformant une première image de tomodensitométrie transformée, qui a été reconstruite sur la base d'un ensemble de projections mesurées et qui a ensuite été transformée, de sorte qu'elle correspond à une autre première image de tomodensitométrie transformée, qui a été reconstruite sur la base d'un autre ensemble de projections mesurées et qui a ensuite été transformée, dans lequel l'unité de reconstruction (13) est conçue pour reconstruire la seconde image de tomodensitométrie tridimensionnelle à mouvement corrigé également sur la base de la transformation supplémentaire.

**12.** Appareil de production d'image de tomodensitométrie comme défini dans la revendication 10, dans lequel l'unité de reconstruction (13) est conçue pour diviser les projections mesurées en plusieurs ensembles de projections mesurées de sorte qu'au moins un ensemble de projections mesurées ne satisfait pas au critère d'exhaustivité pour la reconstruction de tomodensitométrie.

**13.** Appareil de production d'image de tomodensitométrie comme défini dans la revendication 1, dans lequel l'unité de reconstruction (13) et l'unité de détermination de transformation (14) sont conçues de sorte que, après que la seconde image de tomodensitométrie a été reconstruite, dans une étape itérative les transformations sont de nouveau déterminées sur la base de la seconde image de tomodensitométrie, qui a alors la fonction de la première image de tomodensitométrie, et la seconde image de tomodensitométrie est de nouveau reconstruite sur la base des transformations nouvellement déterminées et des projections mesurées.

**14.** Procédé de production d'image de tomodensitométrie pour produire une image d'une tête humaine, dans lequel le procédé de production d'image de tomodensitométrie comprend :

- la fourniture de projections bidimensionnelles mesurées de la tête par une unité de fourniture de projections (12), dans lesquelles les projections mesurées ont été mesurées à des instants différents tandis qu'une source de rayonnement (2), qui émet un rayonnement (4) pour traverser la tête, a été déplacée autour de la tête et dans lesquelles les projections mesurées ont été produites sur la base du rayonnement (4) après avoir traversé la tête,
- la reconstruction par une unité de reconstruction (13) d'une première image de tomodensitométrie tridimensionnelle (15) de la tête sur la base des projections mesurées fournies,
- la détermination de transformations tridimensionnelles de la première image de tomodensitométrie de la tête pour des groupes de projections mesurées différents par une unité de détermination de transformation (14), dans laquelle un groupe de projections mesurées comprend une ou plusieurs projections mesurées, dans laquelle l'unité de détermination de transformation (14) détermine pour un certain groupe de projections mesurées une transformation de sorte qu'un degré de similitude entre le certain groupe de projections mesurées et un groupe de projections calculées (16) est augmenté,

dans lequel le groupe de projections calculées (16) correspond au certain groupe de projections mesurées et est calculé en transformant la première image de tomodensitométrie (15) selon la transformation à déterminer et par projection en avant de la première image de tomodensitométrie transformée (15),
dans lequel l'unité de reconstruction (13) reconstruit une seconde image de tomodensitométrie tridimensionnelle à mouvement corrigé sur la base des projections mesurées et des transformations déterminées pour les groupes de projections mesurées différents, dans lequel l'appareil de production d'image de tomodensitométrie (10) comprend une zone d'examen dans laquelle la tête humaine peut être agencée, dans lequel l'unité de reconstruction (13) reconstruit la première image de tomodensitométrie et la seconde image de tomodensitométrie de sorte qu'elles montrent la zone d'examen et afin de, pour produire la seconde image de tomodensitométrie, effectuer la correction de mouvement pour une première partie de la zone d'examen et ne pas effectuer la correction de mouvement pour une seconde partie de la zone d'examen, dans lesquelles la première partie de la zone d'examen inclut la tête humaine.

**15.** Programme informatique pour commander un appareil de production d'image de tomodensitométrie comme défini dans la revendication 1, le programme informatique comprenant un moyen formant code de programme pour amener l'appareil de production d'image de tomodensitométrie à effectuer les étapes du procédé de production d'image de tomodensitométrie comme défini dans la revendication 14, quand le programme informatique est exécuté sur un ordinateur commandant l'appareil de production d'image de tomodensitométrie.

# FIG. 1

FIG. 2

FIG. 3

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **C. BODENSTEINER et al.** Motion and Positional Error Correction for Cone Beam 3D-Reconstruction with Mobile C-Arms. *Medical Image Computing and Computer-Assisted Intervention,* 2007, vol. 4791, 177-185
- **JIANG HSIEH.** Computed Tomography Principles, Design, Artifacts, and Recent Advances. John Wiley & Sons, 2009, 264-266
- **I.M.J. VAN DER BOM et al.** Evaluation of optimization methods for intensity-based 2D-3D registration in x-ray guided interventions. *SPIE Medical Imaging,* 2011
- **P. GRANGEAT et al.** Theoretical framework for a dynamic cone-beam reconstruction algorithm based on a dynamic particle model. *Physics in Medicine and Biology,* 2002, vol. 47, 2611-2625
- **L. DESBAT et al.** Compensation of Some Time Dependent Deformations in Tomography. *IEEE Transactions on Medical Imaging,* 2007, vol. 26 (2), 261-269
- **S. BONNET et al.** Dynamic X-Ray Computed Tomography. *Proceedings of the IEEE,* 2003, vol. 91 (10), 1574-1587